Europäisches Patentamt

**European Patent Office**

Office européen des brevets

(11) Publication number: **0 153 200**
A2

# EUROPEAN PATENT APPLICATION

(21) Application number: 85301163.3

(22) Date of filing: **21.02.85**

(51) Int. Cl.⁴: **A 61 L 15/03,** A 61 K 9/70,
A 61 K 47/00

(30) Priority: **21.02.84 JP 31935/84**

(71) Applicant: **YAMANOUCHI PHARMACEUTICAL CO. LTD.,
No. 5-1 Nihonbashi-Honcho, 2-chome Chuo-ku, Tokyo
(JP)**

(43) Date of publication of application: **28.08.85**
**Bulletin 85/35**

(72) Inventor: **Konno, Yutaka, No. 21-6, Bessho-cho,
Ohmiya-shi Saitama (JP)**
Inventor: **Kawata, Hiroitsu, No. 1-7, Ohaza Namiki,
Kawagoe-shi Saitama (JP)**
Inventor: **Aruga, Masayoshi, No. 16-14,
Midorigaoka 5-chome, Ageo-shi Saitama (JP)**
Inventor: **Sonobe, Takashi, No. 10-12, Fujimi 4-come
Fukiage-cho, Kitaadachi-gun Saitama (JP)**
Inventor: **Mitomi, Mitsuo, No. 23-2, Ohyata 1-chome,
Adachi-ku Tokyo (JP)**

(84) Designated Contracting States: **DE FR GB IT**

(74) Representative: **Geering, Keith Edwin et al, REDDIE &
GROSE 16 Theobalds Road, London WC1X 8PL (GB)**

(54) **Medicinal patch.**

(57) This invention provides a medicinal patch comprising a
solution or suspension of drug component and penetration
enhancer dispersed in a base selected predominantly from
cacao butter, isocacao butter and triglycerides of $C_{12}$ to $C_{18}$
vegetable saturated fatty acids. This patch composition is
usually provided in a shallow dish for adhering to the skin.

## MEDICINAL PATCH

This invention relates to a patch for transdermal drug administration.

A patch is one form of the transdermal formulations for systemic therapy recently in the limelight, and has hitherto been applied to nitroglycerin, scopolamine, etc.

Known nitroglycerin patches include those containing cross-linked silicone polymer as a base, those containing viscous silicone liquid as a base together with a multi-pore membrane for controlled drug release, and those containing a matrix of polyvinyl alcohol, polyvinyl pyrrolidone, and glycerol as a base. A known scopolamine patch contains a mixture of high molecular polyisobutene, low molecular polyisobutene, and mineral oil as a base.

This invention provides a patch of good transdermal property which uses a suppository base not previously known as a base for patches; it uses fats and oils mainly composed of cacao butter, isocacao butter, and/or triglyceride of vegetable saturated fatty acid having 12 to 18 carbon atoms as a base together with penetration enhancer.

Among the base components, cacao butter and isocacao butter are natural vegetable saturated fatty acid triglycerides, and the latter fats and oils, i.e. the triglycerides of vegetable saturated fatty acids of 12 to 18 carbon atoms, are semi-synthesized ones. The latter fats and oils are the triglycerides of vegetable saturated fatty acids such as lauric acid (C 12), myristic acid (C 14), palmitic acid (C 16), and stearic acid (C 18) and mixtures thereof. They are commercially available under the trade name "Witepsol" (made by Dinamit Nobel Co.). Suitable commercially available fats and oils for the base in this invention include Witepsol H-5, Witepsol H-15, etc.

Thus, according to this invention, there is provided a patch comprising a solution or suspension of drug component and penetration enhancer uniformly dispersed in a base selected at least mainly from cacao butter, isocacao butter and the triglycerides of $C_{12}$ to $C_{18}$ vegetable saturated fatty acids.

Suitable drug components for the patches of this invention include nicardipine hydrochloride, nifedipine, dipyridamole, formoterol fumarate, indenolol, $\gamma$-oryzanol, digoxin, ß-methyldigoxin, dimemorfan phosphate, propranolol, etc. . These drug components have hitherto been supplied as formulations for oral administration and the invention makes their percutaneous administration possible for the first time. The bioavailability of drugs applied to the skin is usually rather low and hence systemic transdermal formulations have been utilized for only a limited number of drugs; also, since transdermal formulations are stuck on the skin, there are restrictions as to the kinds and amounts of base which can be used and as to the additives which can be included whilst maintaining the form of the formulation.

As solvent for dissolving or suspending a drug component in this invention, one which is easily released from the oily base and is compatible with water is fundamentally suitable and examples of the solvent are lactic acid, benzyl alcohol, N-methyl-2-pyrrolidone, crotamiton, glycols (e. g., propylene glycol, ethylene glycol, etc.,), alcohols (e. g., ethanol, isopropanol, etc.;), etc. These solvents may be used alone or in admixture.

The solvent, optionally also providing or in admixture with penetration enhancer, should be selected according to characteristics of the drug component. For nicardipine hydrochloride, an aqueous urea solution, lactic acid, ethanol, propylene glycol, benzyl alcohol, etc., are suitably used in various mixtures, and particularly suitable solvents are a mix of an aqueous urea solution with lactic acid and/or propylene glycol and a mix of an aqueous urea solution, benzyl alcohol, and propylene glycol. A mix of, for example, N-methyl-2-pyrrolidone and propylene glycol is suitably used for nifedipine; a mix of, for example, lactic acid, propylene glycol, and an aqueous urea solution for dipyridamole; a mix of, for example, ethylene glycol and propylene glycol for formoterol fumarate; a solvent such as N-methyl-2-pyrrolidone for $\gamma$-oryzanol; and a solvent such as ethylene glycol or propylene glycol for indenolol.

The amount of solvent used is sufficient for dissolving or suspending the drug component while maintaining the form of the patch.

As penetration enhancer, urea, diisopropyl adipate, diethyl adipate, diethyl sebacate, isopropyl myristate, isopropyl palmitate, middle chain fatty acid glyceride, sorbitan middle chain fatty acid ester, etc., can be used. These penetration enhancers can be used alone or in combination. Since propylene glycol is a solvent and also has a penetration enhancing action, additional penetration enhancer is not necessarily required when it is used as solvent.

The patches of this invention may further contain, in addition to the above-described components, emulsifiers (e. g., a glycerol monostearic acid ester (Nikkol MGS-B, trade name), polyoxyethylene-hardened castor oil (Nikkol HCO-60, trade name), etc.,), hardening agents (e. g., higher alcohols such as cetyl alcohol, etc.,), oily ointment bases (e. g., white petrolatum etc.,),suspending agents (e. g., silicic anhydride (Aerosil, trade name), etc.,), etc., but fundamentally, the base is used in an amount to maintain the patch material in the solid state at normal temperatures.

Especially when the patch material does not melt or soften sufficiently merely on contact with the skin, the patch according to the invention may be provided with heating means, e.g. at its side away from that to be against the skin. Additionally, or instead, the patch according to the invention may have, at its face to be against the skin, an absorbant layer (e.g. filter paper), preferably pre-impregnated with solvent and/or penetration enhancer (e.g. propylene glycol). The patch according to the invention will usually be provided in a shallow dish or like container, preferably

6    0153200

with a protective liner disposed removably across the face to be applied to the skin.

It is preferred that the patch of this invention be prepared by kneading a solution of drug and penetration enhancer with the base. Suitably, with the base molten, e.g. at about 40°C, a drug solution is added gradually while kneading the molten base, and the resultant mixture is kneaded and solidified to provide the patch.

The proportion of each of the patch components of this invention is suitably selected and the amount of the drug component is usually determined according to the nature thereof. Preferably the amount of solvent is the minimum amount capable of dissolving or suspending the drug component. The proportion of base is e.g. 60 to 85 w/w%, the proportion of penetration enhancer is e.g. 0.5 to 20 w/w%, preferably 5 to 15 w/w%, and the total proportion of optional component or components (e.g., emulsifier, suspending agent, preservative, etc.,) is e.g. 0.5 to 10 w/w%.

The patch of this invention can be very easily prepared as compared with the above-described conventional patches, and the base which it uses melts at about body temperature and gives good percutaneous absorption.

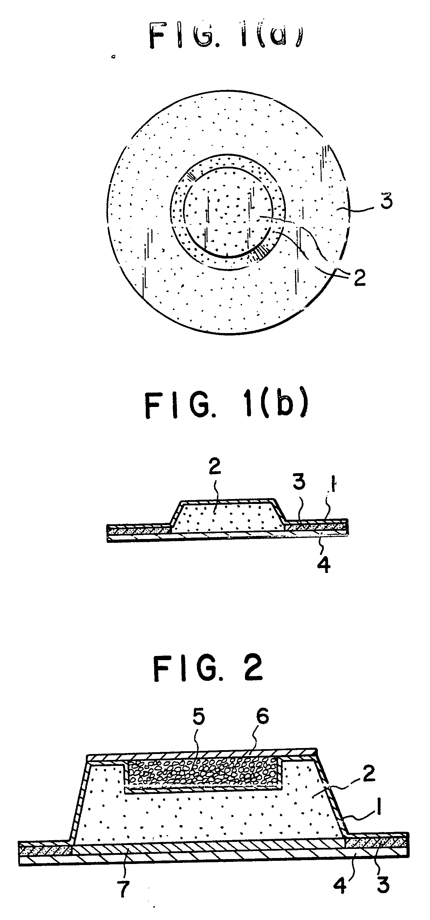

The construction of the patch of this invention may be similar to that of conventional patches. Fig.1 shows the fundamental structure of one example of a patch of this invention, Fig.1(a) being a plan view of the patch and Fig.1(b) a cross sectional view of it. As shown in Fig.1, patch components 2 are packed in a polyvinyl chloride container 1, an adhesive layer 3 is formed at the portion to be applied to the skin, and the surface of the adhesive layer 3 is covered by a liner 4.

Fig.2 is a cross-sectional view, similar to Fig.1(b), of another embodiment of the patch of this invention, having heating means and a filter paper impregnated with propylene glycol.

To improve its form-maintaining property, the patch of this invention sometimes contains a hardening agent such as a higher alcohol (e. g., cetyl alcohol, stearyl alcohol, etc.,), solid paraffin, microcrystalline wax, etc. In this case, however, the melting point range of the patch is liable to become broader and it sometimes happens that the patch is not sharply melted by body heat alone. In such a case, as shown in Fig. 2, a recess at the outer portion (the opposite side to the side to be applied to the skin) of the patch may have heating means 5 disposed therein for heating the patch on application thereof to the skin.

For the heating means a composition mainly composed of iron powder and generating heat in the presence of oxygen and water is used. The upper portion of the heating means is covered by an aluminum seal 6, and the cover is peeled off prior to use. Also, a propylene glycol-impregnated filter paper 7, for example Toyo Filter Paper No. 7 (trade name), pre-impregnated with propylene glycol, is disposed between the patch components 2 and a liner 4. By the foregoing construction, the solution of drug dispersed in the oily base of the patch is attracted to the filter paper, the escape of molten liquid is prevented, and all of the surface area of the patch can be utilized effectively.

9

0153200

The following Examples will still further illustrate this invention.

### Example 1

A mixture of 150.5 g of Witepsol H-15 and 5 g of Nikkol MGS-B was melted at about 60°C and after cooling to about 45°C, the mixture was transferred into a kneader. A solution of 4.5 g of nicardipine hydrochloride in 40 g of a mixture of benzyl alcohol/propylene glycol/aqueous 50% urea solution (1:1:2, w/w) was gradually added to the mixture and the resultant mixture was kneaded. Then the mixture was filled into a polyvinyl chloride container and solidified by cooling to provide a patch.

### Example 2

By following the same procedure as Example 1 using Witepsol H-5 in place of Witepsol H-15, a patch was obtained.

### Example 3

A mixture of 159.1 g of Witepsol H-5 and 9.1 g of Nikkol MGS-B was melted at about 60°C and after cooling to about 45°C, the mixture was transferred into a kneader . Then, a solution of 4.5 g of nicardipine hydrochloride in 27.3 g of a mixture of lactic acid/aqueous 50% urea solution (1 : 2 w/w) was gradually added to the mixture and the resultant mixture was kneaded. The mixture was filled into a polyvinyl chloride container and solidified by cooling to provide a

0153200

patch.

## Example 4

A mixture of 146.4 g of Witepsol H-5 and 9.1 g of Nikkol MGS-B was melted at about 60 °C and after cooling to about 45°C, the mixture was transferred into a kneader.  Then, a solution of 4.5 g of nicardipine hydrochloride ·          in 40 g of a mixture of lactic acid/propylene glycol/aqueous 50% urea solution (1 : 1 : 2 w/w) was gradually added to the mixture and the resultant mixture was kneaded.  The mixture was  filled  into a polyvinyl chloride container and solidified by cooling to provide a patch.

## Example 5

A mixture of 115.5 g of Witepsol H-5, 10 g of Nikkol MGS-B, 10 g of Nikkol TS-10(polyoxyethylene sorbitan monostearate), 10 g of Kalcol 60[ trade name, made by Kao Atlas Co. (palmityl alcohol)]and 10 g of isopropyl myristate was melted at about 60°C and after cooling to about 45°C, the molten mixture was transferred into a kneader.

A    solution of 4.5 g of nicardipine hydrochloride in 40 g of a mixture of benzyl alcohol/propylene glycol/aqueous 50% urea solution (1 : 1 : 2 w/w) was gradually added to the mixture and the resultant mixture was kneaded.  The mixture was filled  into a polyvinyl chloride container and        solidified by cooling to provide a patch.

## Example 6

Heating means was incorporated in the patch of Example 5.  As the heating means, Panakairo 25 (trade name, made by Atlas Shoji K. K.) which is composed of iron powder (main component), activated carbon, sodium chloride, etc., was used.

### Example 7

A mixture of 155 g of Witepsol H-15 and 10 g of Nikkol HCO-60 was melted at about 50°C and after cooling to about 45°C, the molten mixture was transferred into a kneader. Then, a solution of 5 g of nifedipine in a mixture of 10 g of N-methyl-2-pyrrolidone, 10 g of propylene glycol, and 10 g of diisopropyl adipate was gradually added to the mixture and the resultant mixture was kneaded. The mixture was filled into a polyvinyl chloride container and solidified by cooling to provide a patch.

### Example 8

A mixture of 148.65 g of Witepsol H-5 and 9.1 g of Nikkol MGS-B was melted at about 60°C and after cooling to about 45°C, the molten mixture was transferred into a kneader. Then, a solution of 2.25 g of dipyridamole in 40 g of a mixture of lactic acid/propylene glycol/aqueous 50% urea solution (1 : 1 : 2 w/w) was gradually added to the mixture and the resultant mixture was kneaded. The mixture was filled into a polyvinyl chloride container and solidified by cooling to provide a patch.

### Example 9

A mixture of 160 g of Witepsol H-15 and 10 g of Nikkol MGS-B was melted at about 60°C and after cooling to about 45°C, the molten mixture was transferred into a kneader. Then, a solution of 10 mg of formoterol

0153200

fumarate in 30 g of a mixture of ethylene glycol/aqueous 50% urea solution (1 : 2 w/w) was gradually added to the mixture and the resultant mixture was kneaded. The mixture was filled into a polyvinyl chloride container and solidified by cooling to provide a patch.

### Example 10

A mixture of 157.5 g of Witepsol H-15 and 10 g of Nikkol MGS-B was melted at about 60°C and after cooling to about 45°C, the molten mixture was transferred into a kneader. Then, a solution of 2.5 g of indenolol in 30 g of a mixture of ethylene glycol/aqueous 50% urea solution (1 : 2 w/w) was gradually added to the mixture and the resultant mixture was kneaded. The mixture was treated as in Example 9 to provide a patch.

### Example 11

A mixture of 165 g of Witepsol H-5 and 10 g of Nikkol MGS-B was melted at about 60°C and after cooling to about 45°C, the molten mixture was transferred into a kneader. Then, a solution of 5 g of $\gamma$-oryzanol in 20 g of a mixture of N-methyl-2-pyrrolidone/diisopropyl adipate (1 : 1 w/w) was gradually added to the solution and the resultant mixture was kneaded. Then, the mixture was treated as in Example 10 to provide a patch.

### Comparison Example

A mixture of 155.5 g of Witepsol H-5, 10 g of

0153200

Nikkol MGS-B, 10 g of Nikkol IPM (isopropyl myristate), 10 g of Nikkol TS-10 and 10 g of Kalcol 60 was melted and after cooling to about 45°C, the mixture was transferred into a kneader. Then, 4.5 g of nicardipine hydrochloride was added to the mixture followed by kneading and the mixture thus obtained was filled into a polyvinyl chloride container and solidified to provide a patch.

Percutaneous absorption test in guinea pigs

Patches of about 3 g, one each as obtained in Examples 3,4,5 and 6 and the Comparison Example, were applied to the back skin of respective guinea pigs shaved the preceding day; five-fold gauzes,circularly punched out at the centre to fit around the outer diameter of the plastics container,were placed around the plastics container of each patch. The upper surface of each patch thus surrounded by the gauzes was covered by Parafilm and patches were further fixed by surgical tape. 5 hours after application of the patch, blood was collected and the concentration of nicardipine hydrochloride in plasma was measured according to Higuchi et als' method (Journal of Chromatography, 110, 301[1975]). The results thus obtained were as follows :

| Patch | Concentration of nicardipine hydrochloride in plasma |
|---|---|
| Example 3 | 54.0 ng/ml |
| Example 4 | 60.7 " |
| Example 5 | 30.6 " |
| Example 6 | 51.5 " |
| Comparison Example | 5.6 " |

0153200

C L A I M S :

1.    A medicinal patch comprising a solution or suspension of drug component and penetration enhancer dispersed in a base selected predominantly from cacao butter, isocacao butter and triglycerides of $C_{12}$ to $C_{18}$ vegetable saturated fatty acids.

2.    A patch according to claim 1 wherein the drug component is selected from nicardipine hydrochloride, nifedipine, dipyridamole, formoterol  fumarate, indenolol, and γ-oryzanol.

3.    A patch according to claim 1 or 2 containing a solution of nicardipine hydrochloride in a mixture of aqueous urea solution with lactic acid and/or propylene glycol.

4.    A patch according to claim 1 or 2 containing a solution of dipyridamole in a mixture of aqueous urea solution, lactic acid and propylene glycol.

5.    A patch according to claim 1 or 2 containing a solution of nicardipine hydrochloride in a mixture of aqueous urea solution, benzyl alcohol, and propylene glycol.

6.   A patch according to any preceding claim having a propylene glycol-impregnated filter paper disposed at the side of the patch to be applied to the skin.

7.   A patch according to any preceding claim wherein heating means is disposed at the side of the patch opposite to the side thereof to be applied to the skin.

# FIG. 1(a)

# FIG. 1(b)

# FIG. 2